# EUROPEAN PATENT APPLICATION

(11) **EP 2 997 968 A1**
(43) Date of publication of application: **23.03.2016**
(21) Application number: 14386023.7
(22) Date of filing: 22.09.2014
(51) Int. Cl.: A61K 31/295, A61K 31/7004

(54) **Iron (III) hydroxide complexes with activated glucose syrups and process for preparing same**

(71) Applicant: Tseti, Ioulia, 145 61 Kifissia (GR)
(72) Inventor: Tseti, Ioulia, 145 61 Kifissia (GR)
(74) Representative: Wibbelmann, Jobst

(57) **Abstract**

The present invention generally relates to iron (III) carbohydrate complexes and to processes for the manufacture thereof. The product obtainable according to the method of the present invention may be safely used to the general population or animals in the therapy of iron deficiency. The process of the invention includes the steps of (i) providing an aqueous solution of glucose syrup having a certain dextrose equivalent (DE), (ii) adding one or more oxidizing bleaching agents, thereby obtaining the activated glucose syrup; (iii) converting said activated glucose syrup into a complex with iron (III) hydroxide; and (iv) obtaining a complex of iron (III) hydroxide and activated glucose syrup.

## Description

The present invention generally relates to iron (III) carbohydrate complexes and to processes for the manufacture thereof. The product obtainable according to the method of the present invention may be safely used to the general population or animals in the therapy of iron deficiency.

### BACKGROUND OF INVENTION

Iron (III) hydroxide carbohydrate complexes can be produced by reacting suitable carbohydrates with a solution of ferric salts and excess alkali.

It is known that by heating an aqueous solution of dextrin or dextran together with a water soluble ferric salt and alkali at a pH of about 2.3 a perceptible iron complex results which can be depolymerized by hydrolysis to the molecular size desired, and, following this, can be converted by treatment with excess alkali into an iron dextran complex, or, if necessary, can be subjected without depolymerization to the treatment with alkali directly (see US 2,885,393, or US 3,076,798).

GB 1076219 refers to parenteral iron preparations for the prophylaxis or treatment of iron deficiency anemia. Described is a method for the manufacture of a complex containing iron and low molecular weight dextrin or dextran with sorbitol. A non-ionic iron-carbohydrate complex is formed with ferric hydroxide and a complex-forming agent consisting of a mixture of sorbitol (about 0.4 mol), gluconic acid (about 0.3 mol) and a polyglucose (about 0.3 mol), the polyglucose comprising dextrin, dextran, hydrogenated dextrin or hydrogenated dextran having intrinsic viscosity 0.01-0.025 at 25°C, and average molecular weights 500-1200. The hydrogenated polyglucoses are substantially non-reducing to Somogyi reagent. The complex is made by treating 1 mol of a trivalent iron compound in aqueous solution with about 2 mols of complex-forming agent having the molar ratio of sorbitol: gluconic acid: polyglucose about 1.15: 0.40: 0.5, and heating the mixture at an alkaline pH.

US 2,885,393 discloses a complex of iron and dextran formed by interaction of a water soluble ferric salt and dextran whereby said complex is formed. Following formation of the complex, it may be isolated by addition of a water-soluble organic solvent such as a lower alcohol, ketone, glycol, mixtures thereof, or the like. Preferably volatile solvents such as the lower alkanols are employed, since this facilitates subsequent elimination. The precipitated complex can be purified by successive dissolutions in water followed by precipitations with alcohol or the like. In addition, the solution of the complex may be heated to partially degrade the dextran and alkali subsequently added to render the solution highly alkaline. Any unreacted iron will then be taken up by the dextran. The solution can then be neutralized and the dextran complex isolated. The isolated complex is then dissolved in water to form a stock solution which can be brought to any desired concentration. As ferric salts there may be employed any water-soluble salts such as ferric chloride, nitrate, sulfate, acetate, or the like. The specific anion is not material since it does not enter into the reaction. Suitable alkalies include alkali metal hydroxides, ammonium hydroxide, tetramethyl ammonium hydroxide, and the like, as well as the carbonates and bicarbonates of these alkalies, although any water-soluble alkalies may be similarly employed.

US 4,927,756 discloses a water-soluble iron dextran having a high iron content, which is prepared by reacting dextran, having an average molar mass of from 2000 to 4000, with freshly precipitated iron(III) hydroxide and, if desired, further purifying the same. Specifically disclosed are iron dextrans having an iron content of from 27 to 33 percent by weight and an average molar mass of the dextran component of from 2000 to 4000.

US 3,076,798 discloses a process of producing an iron injection preparation which is suitable for parenteral medication for the treatment of iron deficiency anemia in humans and animals. The ferric hydroxide-polymaltose complex is formed by heating the mixture of a water-soluble dextrin and an aqueous solution containing ferric ions and an excess of an alkali hydroxide or an alkali carbonate to a temperature of from 60° to 100°C.

US 3,908,004 discloses a method of making an iron-containing composition to be injected for the treatment of iron-deficiency anaemia. In carrying out the method, a monosaccharide or an oligosaccharide is polymerised and the polymerised product is heated with an aqueous alkali and the mixture is separated into two or more fractions of different molecular weight. A fraction is then selected containing the desired polysaccharide and these are reacted with a water soluble inorganic iron compound.

US2013/0203698 A1/WO20040378 (A1) discloses water-soluble iron carbohydrate complexes, prepared by oxidizing maltodextrins by use of e.g. hypochlorite.

EP1554315 B1 and EP2287204, of the same patent family like US2013/0203698 A1, also disclose a water-soluble iron-carbohydrate complex obtained from an aqueous iron (III)-salt solution and an aqueous solution of the product obtained by oxidizing one or several maltodextrins with an aqueous hypochlorite solution at an alkaline pH value. The dextrose equivalent of the maltodextrin ranges from 5 to 20 if a single maltodextrin is used while the dextrose equivalent of the mixture of several maltodextrins ranges from 5 to 20 and the dextrose equivalent of each individual maltodextrin contained in the mixture ranges from 2 to 40 if a mixture of several maltodextrins is used.

WO 03/087164 discloses an iron-dextrin compound for treatment of iron deficiency anaemia comprising hydrogenated dextrin having a weight average molecular weight equal to or less than 3,000 Dalton and a number average molecular weight equal to or higher than 400 Daltons, in stable association with ferric oxyhydroxide. It furthermore teaches that, as the molecular weight of the dextrin must be narrow, it is an important feature that the 10 % fraction of the dextrins having the highest molecular weight has an average molecular weight of less than 4500 Daltons, and that 90 % of the dextrins are having molecular weights of less than 3000 Daltons. It is further important that the 10 % fraction having the lowest molecular weight has a weight average molecular weight of 340 Daltons or more.

US 4,180,567 also discloses the preparation of a polyhydric compounds by use of sodium borohydride.

EP 1858930 discloses a process for the preparation of trivalent iron complexes with mono-, di- and polysaccharide sugars, consisting of the activation of the sugar by oxidation with nascent bromine generated *in situ* by reaction between an alkaline or alkaline earth bromine and an alkaline hypochlorite, the complexation of the activated sugar in solution with a ferric salt dissolved in an aqueous solution, the purification of the resulting solution through ultrafiltration and finally the stabilization of the trivalent iron-sugar complex by heating at a temperature between 60°C and 100°C for a period between 1 and 4 hours at a pH between 9.0 and 11.0.

Oxidation of glucose syrups with bromine or electrolytic oxidation is reported in Gallali et al. (starch/starke 37 (1985) Nr. 2, pages 58-61).

GB 1,322,102 discloses iron complexes prepared by using polysaccharides which have been modified by oxidation or alkali degradation.

US 5,866,533 and EP0755944 A2 refer to the oxidation of maltodextrins having a dextrose equivalent of below 20.

Although various iron (III) dextrin complexes are known, there is still a need for improved complexes and methods of preparing same. In particular, there is a need for complexes which are stable and, at the same time, provide a high iron content.

### SUMMARY OF THE INVENTION

The present application describes stable iron (III) hydroxide complexes with activated glucose syrups and processes for preparing same. The process for the preparation of complexes of iron (III) hydroxide and activated glucose syrup, said complexes, and pharmaceutical compositions comprising said complexes of the invention are defined in the claims.

The complexes of the present invention are stable and show a surprisingly high stability over a wide range of pH values of from 0 to 14 without any precipitation from a 5% aqueous solution of the product. The products may therefore be used for the therapy of iron deficiency in humans or animals.

### DESCRIPTION OF FIGURES

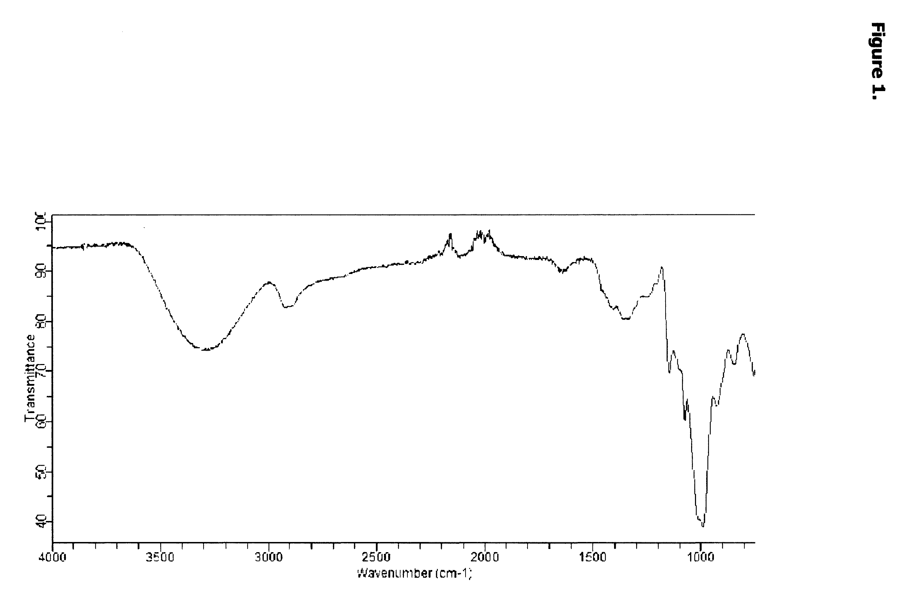

**Figure 1** (FT-IR sugar DE21) shows FT-IR spectrum of glucose syrup with DE21

**Spectrum description:**

| Band (cm⁻¹) | Morphology | Attribution |
|---|---|---|
| 3400 - 3200 | weak, enlarged | O-H |
| 2930 | weak, enlarged | C-H |
| 1640 - 1690 | weak | C=O |
| 1010 | intense, enlarged | C-O |

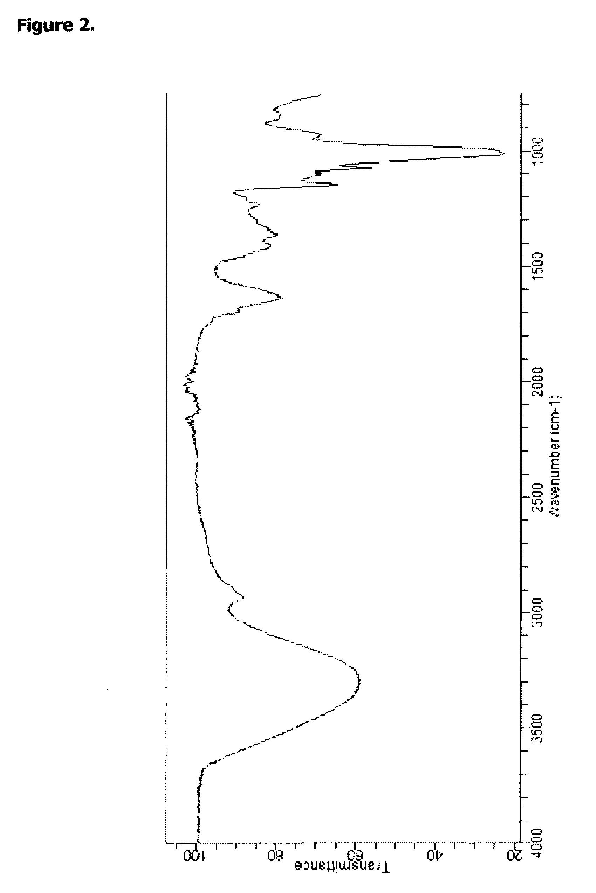

**Figure 2** (FT-IR oxidized sugar DE21) shows FT-IR spectrum of glucose syrup oxidized (method of the invention)

**Spectrum description:**

| Band cm⁻¹ | Morphology | Attribution |
|---|---|---|
| 3400 - 3200 | weak, enlarged | O-H |
| 2960 | weak, enlarged | C-H |
| 1640 - 1690 | medium, enlarged | C=O |
| 1010 | intense, enlarged | C-O |

The differences between these IR spectra (of DE21 and DE21 oxidized) can be easily observed from the intensity of the carbonyl band in 1640-1690 cm⁻¹. So IR spectroscopy is a useful tool to distinguish the oxidized glucose syrups from non-oxidized.

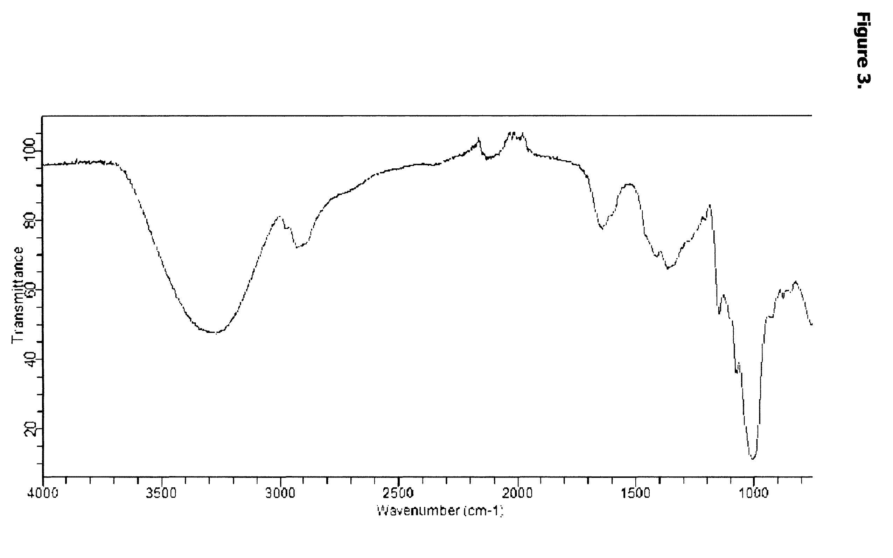

**Figure 3** (FT-IR oxidized sugar Example 1 of US2013/0203698) shows FT-IR spectrum of sugar oxidized according to method of Example 1 described in US2013/0203698.

**Spectrum description:**

| Band (cm⁻¹) | Morphology | Attribution |
|---|---|---|
| 3400 - 3200 | weak, enlarged | O-H |
| 2960 | weak, enlarged | C-H |
| 1640 - 1690 | medium, enlarged | C=O |
| 1010 | intense, enlarged | C-O |

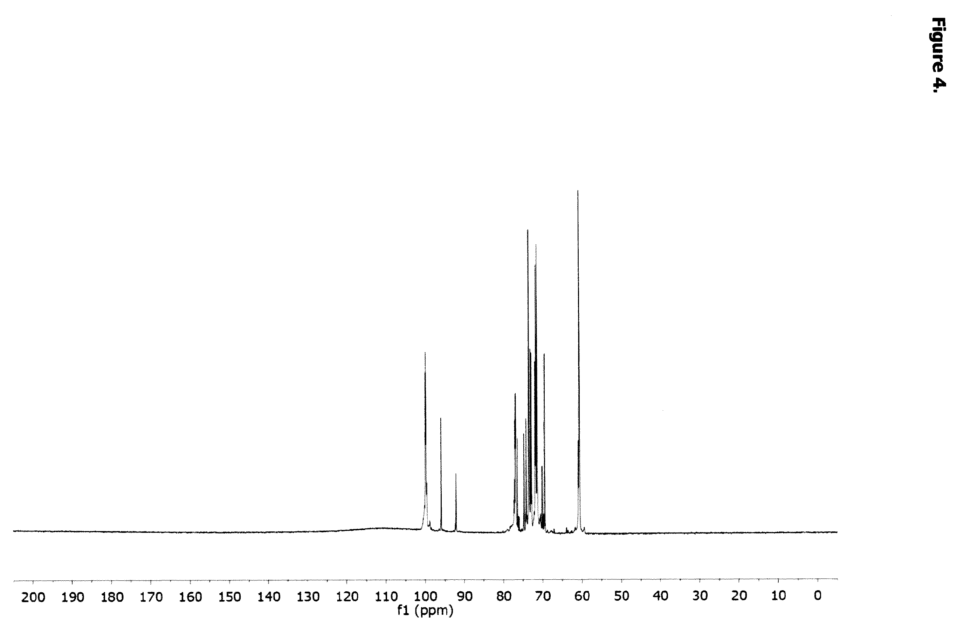

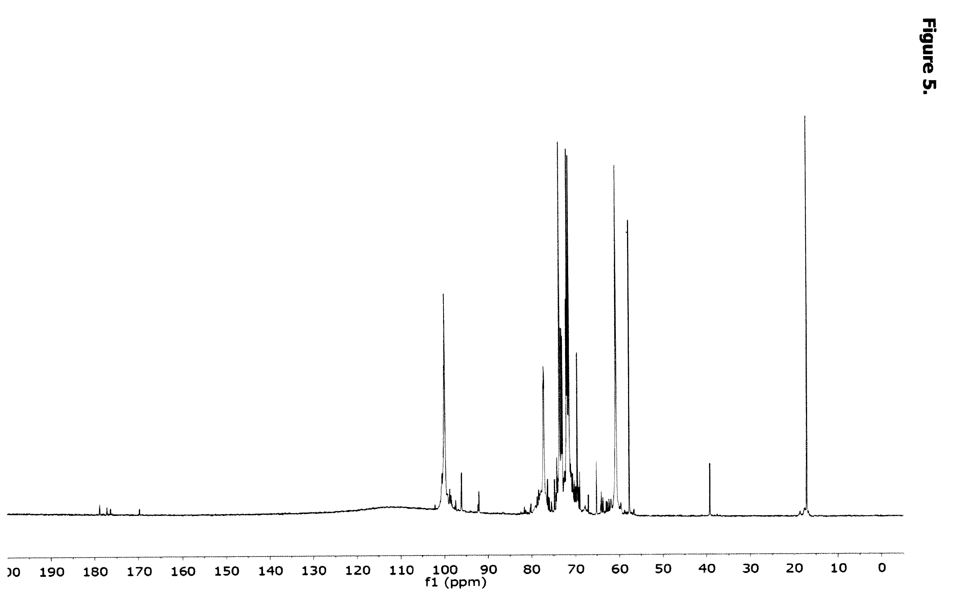

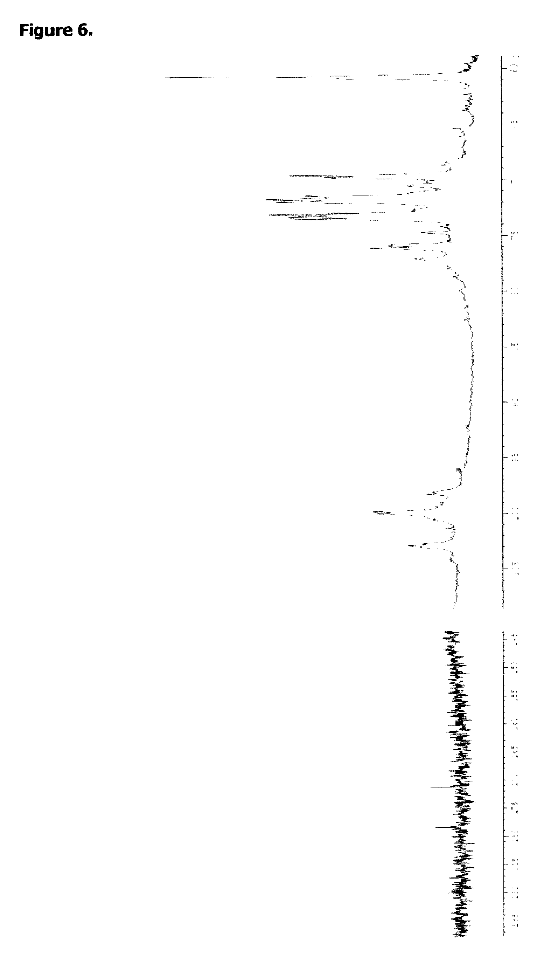

**Figure 4** (¹³C NMR spectrum of sugar (DE21) prior the oxidation) shows ¹³C NMR spectrum of sugar (DE21) prior the oxidation. As it is expected, no chemical shifts are observed at the low field area from 160 to 200 ppm (carbonyl groups) in ¹³C NMR spectrum of the starting sugar. This is presented for comparison reasons to Figure 5 and Figure 6.
**Figure 5** (¹³C NMR spectrum of DE 21 oxidized (present invention)) shows ¹³C NMR spectrum of sugar DE 21 oxidized (present invention). The ¹³C NMR spectroscopy confirms the oxidation of the sugar (DE21) using as oxidative reagent the H₂O₂. There are four distinguished chemical shifts assigned to carbonyl groups at 178.8, 177.2, 176.3 and 169.7 ppm on the oxidized sugar according to the oxidation of the present invention.
**Figure 6** (¹³C NMR spectrum of oxidized sugar according to US2013/0203698, Example 1) shows ¹³C NMR spectrum of oxidized sugar according to patent US2013/0203698 Example 1. This spectrum presents two distinguished chemical shifts assigned to carbonyl groups at 178.4 and 171.2 ppm. This might represent evidence that the oxidation according to the present invention using H₂O₂ is different and gives more oxidized sugar as product at the activation step, in contrast to US2013/0203698 patent Example 1 (oxidant NaClO).
¹³C magnetic resonance spectroscopy conclusions:
The ¹³C NMR spectra (**Figure 5** and **Figure 6**) confirm that the two oxidized sugars (present invention vs Example 1 of US2013/0203698 patent) are structurally different on the carbonyl band (168 - 180 ppm). The oxidized sugar of the present invention can present 4 distinguished carbonyl groups which demonstrates that the degree of oxidation of this sugar is higher than 2 carbonyl groups of the prior art oxidized sugar.
The ¹³C NMR spectra supply spectroscopic evidence that all the examined sugars are oxidized and there is also a distinguish difference between the oxidation process and oxidation products of the present invention in contrast to the prior art oxidation processes and products.

### DETAILED DESCRIPTION OF THE INVENTION

The present invention refers to a process for the preparation of complexes of iron (III) hydroxide and activated glucose syrup comprising, preferably consisting of, the steps:
(i) providing an aqueous solution of glucose syrup, having a dextrose equivalent (DE) of at least 21, as determined by gravimetrical analysis at a temperature in the range of from 25°C to 80°C and at a pH in the range of from 6 to 13;
(ii) adding one or more oxidizing bleaching agents, and optionally a catalytic amount of an oxidation catalyst, to the solution of (i), while maintaining the pH and temperature within the range as defined in step (i), allowing the solution to cool down to a temperature in the range of from 10°C to 45°C, keeping the solution at this temperature for 5 min to 24 hours, thereby obtaining the activated glucose syrup;
(iii) converting said activated glucose syrup into a complex with iron (III) hydroxide; and
(iv) obtaining a complex of iron (III) hydroxide and activated glucose syrup,
wherein the oxidizing bleaching agent(s) is/are used in an total amount of 0.0005-0.01 mol/g (glucose syrup) multiplied with the correction factor: (dextrose equivalent of the glucose syrup)/21, wherein the complex has an iron content in the range of from 27 to 35 wt.%, based on the weight of the complex; and wherein the obtained complex of iron (III) hydroxide and activated glucose syrup has more than two -COOH groups per glucose molecule which indicates that the glucose syrup is activated and wherein the glucose syrup of step (i) does not have any -COOH group per/at the glucose molecule.

The water that is used in step (i) is preferably purified water, which meets the requirements of the current version of the EU Pharmacopoeia at the filing date of the present application. Upon dissolving the glucose syrups, preferably under stirring, a clear solution is obtained. The pH in step (i) is in the range of from 6 to 13, such as 8.5 to 11.5, preferably is in the range of from 10.5 to 11.0. Adjustment of pH in step (i) can be performed by addition of inorganic bases such as aqueous sodium hydroxide (e.g. 40% w/w). The temperature of the solution in (i) is set to a value in the range of from 25°C to 80°C, preferably between 45°C and 60°C, more preferably between 48°C and 55°C. A higher iron content can be obtained when performing step (ii) at a higher pH (see Example 1 and 2). Thus, a pH range of from 10.5 to 13 or from 10.5 to 11.5 is preferred.

The optional oxidation catalyst, such as NaBr, or mixture of oxidation catalysts is used in catalytic amounts such as 50 nanomols (nM) per g of glucose syrup.

In step (ii), the oxidizing bleaching agent is preferably slowly added to the solution of step (i), e.g. stepwise, over a period of time, for example 1 to 4 hours. By way of example, Example 1 as described herein, adds 31 g of a 35% (w/w) aqueous solution of hydrogen peroxide at about 0.25 ml/min.

In step (ii), the glucose syrup is "activated" which means that it is fully oxidized. This means that the present invention oxidizes to a higher extent than the prior art processes. During the addition of the oxidizing bleaching agent, the temperature is kept in the range of from 25°C to 80°C, preferably between 45°C and 60°C, more preferably between 48°C and 55°C.

After the complete amount of oxidizing bleaching agent has been added, the reaction mixture is allowed to react for some time at the same conditions, e.g. for 5 to 15 minutes, and then allowed to cool down to a temperature of 10-45°C and is kept at this temperature for a period of time such as 5 min to 24 hours, e.g. 5 hours, while keeping the pH in the range of from 6 to 13, such as 8.5 to 11.5, preferably in the range of from 10.5 to 11.5, such as 10.65 to 10.85.

Step (iii) of obtaining an activated glucose syrup can include to keep the solution at a temperature in the range of from 18°C to 25°C for period of time of from 1 to 10 hours after cooling down the solution after complete addition of the oxidizing bleaching agent and before use of said activated glucose syrup for preparing the desired complex such as by performing steps (iii)(a)-(iii)(c) below.

The dextrose equivalent (DE) of the activated glucose syrup is preferably in the range of from 0.1 to 1.0, preferably is about 0.3 and/or the obtained complex of iron (III) hydroxide and activated glucose syrup has more than two, preferably more than three - COOH groups per glucose molecule, which indicates that the glucose syrup is activated.

The molecular weight of the complex is preferably in the range of from 50 kDa to 250 kDa, or 100 to 150 kDa, as measured by high performance liquid chromatography-gel permeation chromatography (HPLC-GPC).

The step of converting said activated glucose syrup into a complex with iron (III) hydroxide preferably comprises the steps of:
(iii)(a) adding a solution of FeCl₃ to the solution of (ii) at a temperature within the range of from 10 to 30°C; preferably, the amount of FeCl₃ is in the range of from 30% wt.-% to 120% wt.-% of the amount of glucose syrup.
(iii)(b) adding an inorganic base to the reaction mixture of step (iii)(a)until the pH is within a range of from 1.5 to 2.5; and
(iii)(c) heating the reaction mixture of step (iii)(b) to a temperature within the range of from 40 to 60°C.

To the solution obtained in step (ii), an aqueous solution of FeCl₃ is added in step (iii)(a). The pH of the solution is then adjusted in step (iii)(b) by using inorganic bases such as Na₂CO₃. In step (iii)(c), the reaction mixture is heated for some time, such as 30 minutes to 1 hour, then preferably, the pH is slowly adjusted to 9-12, further preferred 10-11 by using inorganic bases, such as aqueous NaOH. After some time of reaction at a high temperature, preferably in the range of from 50°C to 60°C, for example for 1-3 hours, the reaction mixture is cooled to a temperature in the range of from 18 to 25°C and the pH is then adjusted to be in a range of from 4 to 7, preferably 5-6. The obtained complex can then be purified from the salts by a) ultrafiltration with a cut-off of 30 kDa b) precipitation with ethanol (2:1 to 1:5 / solution: ethanol). The final product can then be isolated, e.g. dried, for example by using a spray drier.

The oxidizing bleaching agents is preferably one or more selected from the group of hydrogen peroxide, ammonium persulfate, sodium and calcium hypochlorite, potassium permanganate and sodium chlorite, most preferably the oxidizing bleaching agent is hydrogen peroxide.

In one embodiment, 0.001-0.003 mol/g of glucose syrup are used. In one embodiment, about 0.0019 mol/ g of glucose syrup, preferably with hydrogen peroxide as the bleaching agent, are used.

The oxidation catalyst preferably includes bromine and iodine ions.

The present invention also refers to a complex of iron (III) hydroxide and glucose syrup obtainable or obtained by the process of the present invention.

The present invention also refers to a pharmaceutical composition comprising a complex of the invention. Said complex or pharmaceutical composition can be used as medicament. Specifically, said complex or pharmaceutical composition can be used in a method of treating iron deficiency in human and animal. The treatment can comprise parenterally administering said complex or pharmaceutical composition.

Dextrins are carbohydrates with low molecular weight, which can be produced by hydrolysis of starch or glycogen. Dextrins are mixtures of polymers of D-glucose units linked by a-(1-4) or a-(1-6) glycosidic bonds.

Dextrins can be produced from starch using enzymes like amylases, as during digestion in the human body and during malting and mashing, or by applying dry heat under acidic conditions (pyrolysis or roasting). During roasting under acid condition the starch hydrolyses and short chained starch parts partially rebranch with a-(1,6) bonds to the degraded starch molecule.

Dextrins are white, yellow, or brown powders that are partially or fully water-soluble, yielding optically active solutions of low viscosity. Most can be detected with iodine solution, giving a red coloration; one distinguishes erythrodextrin (dextrin that colours red) and achrodextrin (giving no colour).

Maltodextrin consists of D-glucose units connected in chains of variable length. The glucose units are primarily linked with a(1→4) glycosidic bonds. Maltodextrin is typically composed of a mixture of chains that vary from three to seventeen glucose units long.

Maltodextrins are classified by DE (dextrose equivalent) and have a DE between 3 an 20. The higher the DE value, the shorter the glucose chains, the higher the sweetness, the higher the solubility and the lower heat resistance.

Above DE 20, the European Union's CN code calls it glucose syrup, at DE 10 or lower the customs CN code nomenclature classifies maltodextrins as dextrins.

Maltodextrin (see formula below) and glucose syrup are polysaccharide that are used as a food additive. They are produced from starch by partial hydrolysis and are usually found as a white hygroscopic spray-dried powders.

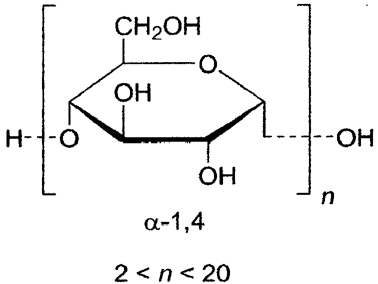

Dextrose equivalent (DE) is a measure of the amount of reducing sugars present in a sugar product, relative to dextrose (a.k.a glucose), expressed as a percentage on a dry basis. For example, a maltodextrin with a DE of 10 would have 10% of the reducing power of dextrose (which has a DE of 100). Maltose, a disaccharide made of two glucose (dextrose) molecules has a DE of 52, correcting for the water loss in molecular weight when the two molecules are combined (180/342). For solutions made from starch, it is an estimate of the percentage reducing sugars present in the total starch product.

Dextrose equivalent (DE) can be measured gravimetrically as described in US 2013/0203698 A1: Dextrins are reacted in a boiling aqueous solution with Fehling's solution. The reaction is carried out quantitatively, i.e. until the Fehling's solution is no longer discoloured. The precipitated copper (I) oxide is dried at 105°C until a constant weight is achieved and measured gravimetrically. The glucose content (dextrose equivalent) is calculated from the obtained results as % weight/weight of the dextrin dry substance. It is, for example, possible to use the following solutions: 25 ml Fehling's solution I, mixed with 25 ml Fehling's solution II; 10 ml aqueous maltodextrin solution (10% m/vol) (Fehling's solution I: 34.6 g copper (II) sulfate dissolved in 500 ml water; Fehling's solution II: 173 g potassium sodium tartrate and 50 g sodium hydroxide dissolved in 500 ml water) (in addition to the aforementioned method for determining DE values, see also "THE UNIFICATION OF REDUCING SUGAR METHOD", L. S. Munson and P.H. Walker, J. Am. Chem. Soc. 28 (6), 663-686 (1906)).

In all glucose polymers, from the native starch to glucose syrup, the molecular chain begins with a reducing sugar, containing a free aldehyde. As the starch is hydrolysed, the molecules become shorter and more reducing sugars are present.

Thus, the DE value describes the degree of conversion of starch to dextrose, wherein the following definitions are used in the contest of the present invention: starch is close to 0, glucose/dextrose is 100 (percent), dextrins vary between 1 and 13, maltodextrins varies between 3 and 20, glucose syrups contain a minimum of 20% reducing sugars, i.e. a DE of 20. The glucose syrups used in the present invention of a DE of at least 21 and a maximum DE of 60.

The DE gives an indication of the average degree of polymerisation (DP) for untreated, i.e. not oxidized, starch sugars. The rule of thumb is DE × DP = 120.

The present invention relates to products comprising iron (III) hydroxide and activated glucose syrups. The activation of the glucose syrups is performed by using oxidizing bleaching agents such as hydrogen peroxide, ammonium persulfate, potassium permanganate etc. The main purpose of the bleaching of glucose syrup is to improve the quality of the carbohydrates and facilitate the production and stability of the complex with iron (III) salts in a later step. Surprisingly, in comparison with the previous art which describes methods for the production of complexes of iron (III) hydroxide with oxidation products of dextrins with hypochlorite, we have found that we can produce, according to the present application, stable complexes by extending oxidation, which surprisingly gives high oxidized glucose syrups and stable iron complexes in contrast with the results of the prior art.

It is well known in the art that the chemistry of hypochlorite oxidation of starches is relatively complex and primarily involves carbons 2, 3 and 6 on a D-glucopyranosyl unit. It is generally agreed that about 25% of the oxidizing reagent is consumed in carbon - carbon splitting while about 75% oxidizes hydroxyl groups.

In the context of the present invention, it has been found that the activation through the bleaching treatment of glucose syrups gives stable complexes with iron (III) hydroxide.

The 5% w/w aquatic solutions of these complexes show beyond all expectations, stability over a wide range of pH and there is no precipitation at a pH in the range of from 0 to 14 at 25°C.

### EXAMPLES

### EXAMPLE 1.

In a glass reactor are added with continuous stirring 168.0 g of dextrin with DE21 in 268.8 g of purified water. The clear solution is heated to 50°C and added 840 mg of sodium bromide. The pH of the solution is adjusted to 10.8 ±0.1 with the addition of a sodium hydroxide solution 40% w/w. In this solution added slowly 31 g of a solution of hydrogen peroxide 35% w/w (0.25 ± 0.02 ml/min) and the pH of the solution remains between 10.65 - 10.90 with the addition of an aquatic solution of sodium hydroxide 40% w/w. During the addition of hydrogen peroxide the temperature remains between 50 and 55°C. After the end of glucose syrup activation the solution is cooled at room temperature and remains for 5 hours in this temperature with regulation of the pH in the range of 10.65 - 10.85.

In this solution is added 296.35 g of a FeCl₃ solution 36.8% w/w with agitation. In this solution is added slowly anhydrous Na₂CO₃ in powder (0.35 to 0.4 g/min) until the pH of the solution reaches the value of 2.4 ± 0.2. The solution is heated to 50°C and remains at this pH with continuous stirring for 30 min. After the end of this period the pH of the solution is adjusted slowly to 10.5 ± 0.2 with an aquatic solution of sodium hydroxide 40% w/w.

The complex of iron (III) hydroxide with the activated glucose syrup is stabilized with the heating of the solution at 67 ± 2°C for 2 hours and then cooled to room temperature. The pH of the solution is brought to 5.5 ± 0.2 and after that the complex is purified from the salts through an ultrafiltration system equipment with a membrane with a cut-off of 30 KDa. The final product is isolated in dry state with the use of a spray drier.

The physical- chemical analysis of the complex is the following:
Average molecular weight: 100 KDa.
Iron (III) content: 31.4 %.

### EXAMPLE 2.

In a glass reactor are added with continuous stirring 168.0 g of dextrin with DE21 in 268.8 g of purified water. The clear solution is heated to 50°C and added 840 mg of sodium bromide. The pH of the solution is adjusted to 8.5 ±0.1 with the addition of a sodium hydroxide solution 40% w/w. In this solution are added slowly 31 g of a solution of hydrogen peroxide 35% w/w ( 0.25 ± 0.02 ml/min) and the pH of the solution remains between 8.40 - 8.60 with the addition of an aquatic solution of sodium hydroxide 40% w/w. During the addition of hydrogen peroxide the temperature remains between 50 and 55°C. After addition of hydrogen peroxide has ended, the solution is cooled to room temperature and remains for 20 hours at this temperature with regulation of the pH in the range of 8.40 - 8.60.

To this solution is added 296.35 g of a FeCl₃ solution 36.8% w/w with agitation. In this solution, at room temperature, is added slowly anhydrous Na₂CO₃ in powder (0.35 to 0.4 g/min) until the pH of the solution reaches the value of 2.4 ± 0.2. The solution is heated to 50°C and remains at this pH with continuous stirring for 30 min. After the end of this period, the pH of the solution is adjusted slowly to 10.5 ± 0.2 with an aquatic solution of sodium hydroxide 40% w/w.

The complex of iron (III) hydroxide with the activated glucose syrup is stabilized with the heating of the solution at 67 ± 2°C for 2 hours and then cooled to room temperature. The pH of the solution is brought to 5.5 ± 0.2 and, after that, the complex is purified from the salts through an ultrafiltration system equipment with a membrane with a cut-off of 30 KDa. The final product is isolated in dry state with the use of a spray drier.

The physical- chemical analysis of the complex is the following:
Average molecular weight: 150 KDa.
Iron (III) content: 29.2%.

### EXAMPLE 3.

In a glass reactor are added with continuous stirring 168.0 g of dextrin with DE21 in 268.8 g of purified water. The clear solution is heated to 50°C and added 840 mg of sodium bromide. The pH of the solution is adjusted to 9.5 ± 0.1 with the addition of a sodium hydroxide solution 40% w/w. In this solution added slowly 31 g of a solution of hydrogen peroxide 35% w/w (0.25 ± 0.02 ml/min) and the pH of the solution remains between 9.40 - 9.60 with the addition of an aquatic solution of sodium hydroxide 40% w/w. During the addition of hydrogen peroxide the temperature remains between 50 and 55°C. After the end of glucose syrup activation the solution is cooled at room temperature and remains for 5 hours in this temperature with regulation of the pH in the range of 9.40 - 9.60.

In this solution is added 296.35 g of a FeCl₃ solution 36.8% w/w with agitation. In this solution is added slowly anhydrous Na₂CO₃ in powder (0.35 to 0.4 g/min) until the pH of the solution reaches the value of 2.4 ± 0.2. The solution is heated to 50°C and remains at this pH with continuous stirring for 30 min. After the end of this period the pH of the solution is adjusted slowly to 10.5 ± 0.2 with an aquatic solution of sodium hydroxide 40% w/w.

The complex of iron (III) hydroxide with the activated glucose syrup is stabilized with the heating of the solution at 67 ± 2°C for 2 hours and then cooled to room temperature. The pH of the solution is brought to 5.5 ± 0.2 and after that the complex is purified from the salts through an ultrafiltration system equipment with a membrane with a cut-off of 30 KDa. The final product is isolated in dry state with the use of a spray drier.

The physical- chemical analysis of the complex is the following:
Average molecular weight: 145 KDa.
Iron (III) content: 30.6 %.

### EXAMPLE 4.

In a glass reactor are added with continuous stirring 168.0 g of dextrin with DE25 in 268.8 g of purified water. The clear solution is heated to 50°C and added 840 mg of sodium bromide. The pH of the solution is adjusted to 8.5 ±0.1 with the addition of a sodium hydroxide solution 40% w/w. In this solution are added slowly 31 g of a solution of hydrogen peroxide 35% w/w ( 0.25 ± 0.02 ml/min) and the pH of the solution remains between 8.40 - 8.60 with the addition of an aquatic solution of sodium hydroxide 40% w/w. During the addition of hydrogen peroxide the temperature remains between 50 and 55°C. After addition of hydrogen peroxide has ended, the solution is cooled to room temperature and remains for 20 hours at this temperature with regulation of the pH in the range of 8.40 - 8.60 in order to have the complete activation of glucose syrup.

To this solution is added 296.35 g of a FeCl₃ solution 36,8 % w/w with agitation. In this solution, at room temperature, is added slowly anhydrous Na₂CO₃ in powder (0.35 to 0.4 g/min) until the pH of the solution reaches the value of 2.4 ± 0.2. The solution is heated to 50°C and remains at this pH with continuous stirring for 30 min. After the end of this period, the pH of the solution is adjusted slowly to 10.5 ± 0.2 with an aquatic solution of sodium hydroxide 40% w/w.

The complex of iron (III) hydroxide with the activated glucose syrup is stabilized with the heating of the solution at 67 ± 2°C for 2 hours and then cooled to room temperature. The pH of the solution is brought to 5.5 ± 0.2 and, after that; the solution is divided in two equal aliquots and the complex in purified from the salts with the following methods:
a) through an ultrafiltration system equipment with a membrane with a cut-off of 30 KDa. The final product is isolated in dry state with the use of a spray drier.
b) Precipitation of the complex with Ethanol in a range of 1:1. The precipitated complex is dried in a vacuum drier at 48 oC.

The physical- chemical analysis of the complex is the following and is independent from the purification method:
Average molecular weight: 110 KDa.
Iron (III) content: 29.8%.

### EXAMPLE 5.

In a glass reactor are added with continuous stirring 168.0 g of dextrin with DE21 in 268.8 g of purified water. The clear solution is heated to 50°C and added 840 mg of sodium bromide. The pH of the solution is adjusted to 9.5 ±0.1 with the addition of a sodium hydroxide solution 40% w/w. In this solution are added slowly 31 g of a solution of hydrogen peroxide 35% w/w (0.25 ± 0.02 ml/min) and the pH of the solution remains between 9.40 - 9.60 with the addition of an aquatic solution of sodium hydroxide 40% w/w. During the addition of hydrogen peroxide the temperature remains between 50 and 55°C. After addition of hydrogen peroxide has ended, the solution is cooled to room temperature and remains for 20 hours at this temperature with regulation of the pH in the range of 9.40 - 9.60 in order to have the complete activation of glucose syrup.

To this solution is added 296.35 g of a FeCl₃ solution 36.8 % w/w with agitation. In this solution, at room temperature, is added slowly anhydrous Na₂CO₃ in powder (0.35 to 0.4 g/min) until the pH of the solution reaches the value of 2.4 ± 0.2. The solution is heated to 50°C and remains at this pH with continuous stirring for 30 min. After the end of this period, the pH of the solution is adjusted slowly to 10.5 ± 0.2 with an aquatic solution of sodium hydroxide 40% w/w.

The complex of iron (III) hydroxide with the activated glucose syrup is stabilized with the heating of the solution at 67 ± 2°C for 2 hours and then cooled to room temperature. The pH of the solution is brought to 5.5 ± 0.2 and, after that; the solution is divided in two equal aliquots and the complex in purified from the salts with the following methods:
a) through an ultrafiltration system equipment with a membrane with a cut-off of 30 KDa. The final product is isolated in dry state with the use of a spray drier.
b) Precipitation of the complex with Ethanol in a range of 1:1. The precipitated complex is dried in a vacuum drier at 48 oC.

The physical- chemical analysis of the complex is the following and is independent from the purification method:
Average molecular weight: 112 KDa.
Iron (III) content: 30.9%.

### Molecular weight determination of iron (III) complexes

In the context of the present invention, the molecular weight of commercial iron carbohydrate complexes was determined by high performance liquid chromatography-gel permeation chromatography (HPLC-GPC), see United States Pharmacopeia (USP) gel-permeation chromatography method, 28 ed., page 1065.

The Ferinject® product (of Vifor Pharma) has a molecular weight of 200 kDa. The Ferrum Hausmann® product of Vifor Pharma (oral solution) is an iron polymaltose complex having a molecular weight of 50 kDa. The Ferrum Hausmann® product of Vifor Pharma (injectable solution) has a molecular weight of 350 kDa. Furthermore, Example 3 of US2013/0203698 A1 was repeated twice and the molecular weight was determined to be 400 kDa and 450 kDa respectively.

**Table 1. Molecular weight and iron content of iron (III) complexes.**

| | Molecular Weight (MW) | Iron content |
|---|---|---|
| **Example 1** | 100 KDa | Assay_{Fe}(_{III}) : 31.4 % w/w |
| **Example 2** | 150 KDa | Assay_{Fe(III)} : 29.2 % w/w |
| **Example 3** | 145 KDa | Assay_{Fe(III)} : 30.6 % w/w |
| **Example 4** | 110 KDa | Assay_{Fe(III)} : 29.8 % w/w |
| **Example 5** | 112 KDa | Assay_{Fe(III)} : 30.9 % w/w |
| US2013/0203698 A1 (Example 3) | 140 KDa (data given in the reference) | Assay_{Fe(III)} : 26.8 % w/w (data given in the reference) |
| US2013/0203698 A1 (Example 3-reworked) | test 1 | test 1 |
| | 400 KDa | Assay_{Fe(III)} : 25.2 % w/w |
| | test 2 | test 2 |
| | 450 KDa | Assay_{Fe(III)} : 25.4 % w/w |
| US2013/0203698 A1 (Example 1-reworked) | > 450 KDa | Assay_{Fe(III)} : 24.6 % w/w |
| US2013/0203698 A1 (Example 2-reworked) | > 450 KDa | Assay_{Fe(III)} : 12.8 % w/w |
| US2013/0203698 A1 (Example 8-reworked) | 450 KDa | Assay_{Fe(III)} : 15.9 % w/w |

A comparison of US2013/0203698 with a complex of the present invention provided the following results:

**Table 2. Differences between US2013/0203698 Example 1 and the present invention.**

| | **Example 1 of** US2013/0203698 | **Example 2 of the present application** |
|---|---|---|
| Oxidative reagent | NaClO 15 % | H₂O₂ 35 % |
| ¹³C NMR | 2 carbonyl groups (-COOH ) | 4 carbonyl groups (-COOH) |
| Sugar DE | 9.6 | 21 |
| Activated sugar DE | 1.1 | 0.3 |
| Complex MW | > 450 kDa | 150 kDa |
| Fe (III) content | 24.6 % | 29.2 % |

### Cited literature

Gallali et al. "Oxidized Glucose Syrup - Production, Parameters and Food Applications", starch/stärke 37 (1985) Nr. 2, pages 58-61.
L. S. Munson and P.H. Walker, "THE UNIFICATION OF REDUCING SUGAR METHOD", J. Am. Chem. Soc. 28 (6), 663-686 (1906).
EP0755944 A2. EP1554315 B1. EP1858930 A1. EP2287204 A1. GB 1,322,102.
GB 1076219. US 3,908,004. US 4,180,567. US 2013/0203698 A1/ WO2004037865 (A1).
US 3,076,798. US 5,866,533. US 4,927,756. US 3,076,798. US 2,885,393.
WO 03/087164.

## Claims

1. Process for the preparation of complexes of iron (III) hydroxide and activated glucose syrup comprising, preferably consisting of, the steps:
(i) providing an aqueous solution of glucose syrup, having a dextrose equivalent (DE) of at least 21, as determined by gravimetrical analysis at a temperature in the range of from 25°C to 80°C and at a pH in the range of from 6 to 13;
(ii) adding one or more oxidizing bleaching agents, and optionally a catalytic amount of an oxidation catalyst, to the solution of (i), while maintaining the pH and temperature within the range as defined in step (i), allowing the solution to cool down to a temperature in the range of from 10°C to 45°C, keeping the solution at this temperature for 5 min to 24 hours, thereby obtaining the activated glucose syrup;
(iii) converting said activated glucose syrup into a complex with iron (III) hydroxide; and
(iv) obtaining a complex of iron (III) hydroxide and activated glucose syrup, wherein the oxidizing bleaching agent(s) is/are used in an total amount of 0.0005-0.01 mol/g (glucose syrup) multiplied with the correction factor: (dextrose equivalent of the glucose syrup)/21, wherein the complex has an iron content in the range of from 27 to 35 wt.%, based on the weight of the complex; and wherein the obtained complex of iron (III) hydroxide and activated glucose syrup has more than two -COOH groups per glucose molecule which indicates that the glucose syrup is activated and wherein the glucose syrup of step (i) does not have any -COOH group at the glucose molecule.

2. The process of claim 1, wherein the dextrose equivalent (DE) of the activated glucose syrup is in the range of from 0.1 to 1.0, preferably is about 0.3 and/or the obtained complex of iron (III) hydroxide and activated glucose syrup has more than two, preferably more than three -COOH groups per glucose molecule, which indicates that the glucose syrup is activated.

3. The process of claim 1 or 2, wherein the molecular weight of the complex is in the range of from 50 kDa to 250 kDa, or 100 to 150 kDa, as measured by high performance liquid chromatography-gel permeation chromatography (HPLC-GPC).

4. The process of any of claims 1-3, wherein the step of converting said activated glucose syrup into a complex with iron (III) hydroxide comprises the steps of:
(iii)(a) adding a solution of FeCl₃ to the solution of (ii) at a temperature within the range of from 10 to 30°C; preferably, the amount of FeCl₃ is in the range of from 30% wt.-% to 120% wt.-% of the amount of glucose syrup.
(iii)(b) adding an inorganic base to the reaction mixture of step (iii)(a)until the pH is within a range of from 1.5 to 2.5; and
(iii)(c) heating the reaction mixture of step (iii)(b) to a temperature within the range of from 40 to 60°C.

5. The process of any of claims 1-4, wherein the oxidizing bleaching agents is one or more selected from the group of hydrogen peroxide, ammonium persulfate, sodium and calcium hypochlorite, potassium permanganate and sodium chlorite, preferably the oxidizing bleaching agent is hydrogen peroxide.

6. The process of any of claims 1-5, wherein the oxidation catalyst includes bromine and iodine ions.

7. Complex of iron (III) hydroxide and glucose syrup obtainable or obtained by the process of any of claims 1-6.

8. Pharmaceutical composition comprising a complex of claim 7.

9. Complex of claim 7 or pharmaceutical composition of claim 8 for use as medicament.

10. Complex of claim 7 or pharmaceutical composition of claim 8 for use in a method of treating iron deficiency in human and animal.

11. The complex or pharmaceutical composition for use according to claim 10, wherein the treatment comprises parenterally administering said complex of claim 7 or pharmaceutical composition of claim 8.
